(19) Europäisches Patentamt / European Patent Office / Office européen des brevets

(11) **EP 2 742 859 A1**

(12) **EUROPÄISCHE PATENTANMELDUNG**

(43) Veröffentlichungstag:
**18.06.2014 Patentblatt 2014/25**

(51) Int Cl.:
*A61B 5/024* (2006.01)     *A61B 5/029* (2006.01)
*A61B 5/22* (2006.01)     *A63B 22/02* (2006.01)
*A63B 22/06* (2006.01)     *A61B 5/11* (2006.01)
*A61B 5/0205* (2006.01)

(21) Anmeldenummer: **13005724.3**

(22) Anmeldetag: **09.12.2013**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **11.12.2012 DE 102012024225**

(71) Anmelder: **Kreative Technologie LWU UG ( haftungsbeschränkt)**
**82279 Eching am Ammersee (DE)**

(72) Erfinder:
• **Tank, Volker**
**82279 Eching am Ammersee (DE)**
• **Tank, Johannes**
**82279 Eching am Ammersee (DE)**

(54) **Verfahren zur Bestimmung von physiologischen Herz- und Körperleistungsparametern**

(57)     Die Erfindung betrifft Verfahren zur Bestimmung von physiologischen Herz- und Körperleistungsparametern von Personen vorzugsweise bei sportlicher Bewegung wie Gehen, Laufen, Radfahren, Schwimmen, Schilanglauf, etc.. Bewegungsparameter, wie z. B. eine Geschwindigkeit v und eine Herzfrequenz $f_H$, werden gemessen. Daraus werden eine Körperleistung $P_K$, eine Herzarbeit $E_H$ (Körperarbeit je Herzschlag) und eine Herzarbeitskapazität $E_{HK}$ (Körperarbeitskapazität je Herzschlag) ermittelt. Die Veränderungen dieser Parameter werden während wiederholter sportlicher Bewegung über eine Zeit T von Wochen bis zu Jahren bestimmt. So werden der Zustand und die Entwicklung von physiologischen Herz- und Körperleistungsparametern von Personen gegebenenfalls lebenslang kontinuierlich erfasst und überwacht. Die Parameter werden aus Messwerten ermittelt, die bei medizinischer Ergometrie, aber auch von tragbaren Sportcomputern beim Breitensport erfasst werden. Einerseits wird so die Analyse medizinischer Untersuchungen erweitert. Andererseits sind damit Messungen von Herz- und Körperleistung bei sportlicher Bewegung im Breitensport ohne fachmedizinische Unterstützung möglich. Regelmäßig bei jedem Training angewandt, kann jeder Übende seine Trainingserfolge unmittelbar selbst messen, erkennen und ihren Fortschritt kontinuierlich überwachen.

Abb. 1

EP 2 742 859 A1

**Beschreibung**

Die Erfindung betrifft Verfahren zur Bestimmung verschiedener, neuer Parameter zur Beurteilung der physiologischen Herz- und Körperleistungsfähigkeit von Menschen (und Tieren, wie z. B. Pferden). Die Parameter werden aus Messwerten ermittelt, die bei medizinischer Ergometrie, aber auch von tragbaren Sportcomputern beim Breitensport erfasst werden. Einerseits wird so die Analyse medizinischer Untersuchungen erweitert. Andererseits ist damit die Messung von Herz- und Körperleistung bei sportlichem Training oder bei sportlicher Bewegung im Breitensport möglich. Regelmäßig wird bei jedem Training gemessen, somit kann der Übende seine Trainingserfolge unmittelbar erkennen und ihren Fortschritt kontinuierlich überwachen. Insbesondere handelt es sich um die neuen Größen einer Herzarbeit, definiert als Körperarbeit je Herzschlag, einer Herzarbeitskapazität, definiert als Herzarbeit je kg Körpergewicht, eines Herzleistungsäquivalents und eines Herzkörperleistungsäquivalents. Außerdem werden eine Körperleistung und ein Körperleistungsäquivalent bestimmt.

Motivation sind die weltweit stetig zunehmenden Herz-Kreislauferkrankungen, u. a. die Koronare Herzerkrankung. Diese sind seit Jahrzehnten die Haupttodesursache in den Industrieländern. Es sind chronische Krankheiten, also in der Regel nicht heilbar. Ihre medizinische Behandlung verursacht den Hauptteil der Kosten im europäischen Gesundheitswesen. Andererseits ist es seit Jahrzehnten wissenschaftlich belegt, dass regelmäßige körperliche Aktivität (Ausdauertraining) Herzkreislauferkrankungen vorbeugt und den Verlauf bereits manifester Erkrankungen signifikant abmildert. Bei Kranken und auch bei Gesunden ist eine statistische Lebensverlängerung proportional zur Intensität des Ausdauertrainings nachgewiesen. Das Training verursacht nur geringe Kosten (Sportkleidung, eventuell Sportgeräte). Es entlastet das Gesundheitswesen, da in der Regel eine medizinische Behandlung überflüssig oder nur eingeschränkt erforderlich ist. Es beugt allen Arten chronischer Krankheiten vor und steigert die Lebensqualität. Das Training muss allerdings regelmäßig und dauerhaft (lebenslang) durchgeführt werden. Bereitschaft, Disziplin und Motivation dazu sind nicht selbstverständlich, vor allem auch, weil der Erfolg des Trainings langfristig angelegt ist und bisher nicht unmittelbar erkennbar ist. Das liegt unter anderem daran, dass gegenwärtig nur unzulängliche Möglichkeiten existieren, eine Herz- und Körperleistung regelmäßig und unter Belastung z. B. während des Ausdauertrainings zu bestimmen. Eine unmittelbar erkennbare Wirkung des regelmäßigen Ausdauertrainings ist die Verringerung der Herzfrequenz, d. h. bei gleicher Körperleistung wird die Herzfrequenz im zeitlichen Verlauf des Trainings stetig geringer. Die je Herzschlag erbrachte Körperleistung nimmt also zu. Dieser Effekt wird bisher bei Messungen im Ausdauersport und auch bei medizinischen Diagnosen nicht umfassend beachtet.

Gegenwärtig wird eine Herzleistung in der Medizin als Blutförderleistung in der Einheit Milliliter Blut pro Herzschlag S [ml/S] gemessen. Ihre Messung ist aufwändig und erfolgt in der Regel nur bei medizinischer Indikation. Sie ist kein direktes Maß für die je Herzschlag vom Körper erbrachte Leistung. In medizinischen Untersuchungslaboratorien wird gegenwärtig mit Ergometern (Laufbändern oder stationären Fahrrädern) die (in der Regel maximale) Körperleistung in Einheiten von MET (Metabolic Equivalent of Task) oder von PWC (Physical Working Capacity, übersetzt: "Physikalische Arbeitskapazität") gemessen. PWC bezeichnet beispielsweise die in Watt pro Kilogramm Körpergewicht angegebene mechanische Leistung eines Menschen bei einer definierten Herzfrequenz (Puls). Üblich ist die Angabe bei einer Herzfrequenz von 170 Schlägen pro Minute, bezeichnet als $PWC_{170}$. Da ältere Menschen einen Puls von 170 nicht erreichen, werden alternativ auch die $PWC_{130}$ (bei einem Puls von 130 Schlägen pro Minute) und die $PWC_{150}$ (bei einem Puls von 150 Schlägen pro Minute) gemessen (und gegebenenfalls auf $PWC_{170}$ extrapoliert). Eine Herzleistung oder eine Herzarbeitskapazität wird aber nicht bestimmt. {*Angesichts der später erläuterten erfinderischen "Herzarbeitskapazität" wird fol-gende Anmerkung gemacht: Die PWC gibt eine Körperleistung je Kilogramm Körpergewicht bei einer bestimmten Herzfrequenz in der Einheit [W/kg] (also Leistung/Gewicht) an. Die physikalische "Leistung" heißt auf Englisch "power", deshalb ist die PWC eigentlich eine Physical Power Capacity, während "work" Arbeit/Energie bedeutet, um die es hier aber nicht geht.*} Die Messungen von MET oder PWC werden nur bei medizinischer Indikation z. B. bei einem Belastungs-EKG (Elektrokardiogram) durchgeführt. Auch dann geschieht es in der Regel nur in großen zeitlichen Abständen von mehreren Monaten, oder nur einmal im Jahr. Die Messung erfordert geschultes Fachpersonal. Heimtrainingsgeräte (Laufbänder, Fahrräder u. a.) verfügen nicht über vergleichbare Messverfahren; siehe dazu: Sascha Härtel, Entwicklung und Analyse walkingbasierter Ausdauertestverfahren im Rahmen der medizinischen Rehabilitation, Karlsruher sportwissenschaftliche Beiträge Bd. 4, Universitätsverlag Karlsruhe, 2007, ISBN 978-3-86644-171-2.

Gegenwärtig werden zur Unterstützung des individuellen, sportlichen Trainings jeder Art vor allem Pulsuhren verwendet. Diese messen die Herzfrequenz und zeigen sie an. In der Regel sind sie Bestandteil eines tragbaren "Sportcomputers". Je nach Ausführung verfügt dieser über mehrere Messeinrichtungen und Datenspeicher für: Zeit, Stoppuhr, Geschwindigkeit, Geländehöhe, Geländeneigung, Position (auch mittels Satellitennavigation, z. B. GPS), Wegstrecke. Messintervalle und Trainingsprogramme sind meist einstellbar. Verschiedene Fitnesstests sind möglich, so wird beispielsweise eine maximal mögliche Herzfrequenz aus dem Ruhepuls (der Herzfrequenz in Ruhe) abgeleitet, oder ein relativer Fitness-Index angegeben (z. B. von "schwach" bis "exzellent"). Mit den ermittelten Parametern kann die Trainingsintensität überwacht und gesteuert werden. Eine quantitative Körperleistung oder eine quantitative Herzleistung werden dabei nicht bestimmt, insbesondere auch nicht im Training (unter Belastung).

Stationäre Heimtrainingsgeräte (Laufbänder, Fahrräder, etc. für den Hausgebrauch oder in Fitnessstudios) verfügen in der Regel über eine Messung und Anzeige der aufgebrachten Leistung des Trainierenden, nicht aber die einer Herzleistung oder Herzarbeit.

Im Jahre 2010 wurde ein "Verfahren und eine Vorrichtung zur Messung körperlicher Leistungsfähigkeit" (DPMA 10 2010 020 752.7) vorgestellt. Dabei werden aus Weg, Zeit, Herzfrequenz und Gewicht eine "spezifische Leistung" und eine "spezifische Herzleistung" und deren Veränderung im Verlauf der Zeit bestimmt.

Als Nachteil der medizinisch definierten Herzleistung wird angesehen, dass es sowohl bei einem Ausdauertraining als auch bei einer Belastungsergometrie einen sehr großen Aufwand erfordert, die Blutförderleistung kontinuierlich zu messen. Deshalb und weil sie auch kein direktes Maß für die Körperleistung ist, wird diese Messgröße zur Erfassung und Beschreibung der Herzleistung bei Ausdauertraining und Belastungsergometrie für ungeeignet gehalten.

Als Nachteil der Leistungsmessungen (MET oder PWC) in medizinischen Untersuchungslaboratorien wird angesehen, dass diese aufwändig sind und nur bei medizinischer Indikation durchgeführt werden. Nachteilig ist insbesondere, dass dabei eine Herzleistung nicht bestimmt wird. Ein weiterer Nachteil ist, dass nur ein momentaner Status erfasst wird. Dieser ist von der Tagesform und anderen Faktoren beeinflusst und gibt deshalb nicht zuverlässig den Leistungsstand wieder. Nachteilig ist auch, dass diese Verfahren zur kontinuierlichen Überwachung eines regelmäßigen Trainings nicht angewendet werden können, und dass sie Laien nicht durchführen können.

Als Nachteil der existierenden Technik (Pulsmesser und tragbare Sportcomputer) zur Unterstützung und Begleitung des Ausdauertrainings wird angesehen, dass in der Regel mit ihr Leistungsmessungen in freier Bewegung nicht möglich sind und insbesondere die quantitative Bestimmung einer Körperleistung, einer Herzleistung und einer Herzarbeitskapazität in freier Bewegung nicht vorgenommen werden kann.

Als Nachteil der einzigen bekannten Technik zur Leistungsmessung bei freier Bewegung (DPMA 10 2010 020 752.7) wird angesehen, dass die ermittelte "Spezifische Herzleistung" zwei Anteile enthält, nämlich sowohl den der Körperleistung als auch den der Herzleistung. Beide wirken in vergleichbarer Weise: die Körperleistung steigt z. B. bei schnellerer Fortbewegung, die Herzleistung ist bei gegebener Körperleistung größer, wenn die Herzfrequenz geringer ist. Da beide Leistungsanteile nicht getrennt werden, ist nicht unmittelbar eindeutig erkennbar, wie groß der jeweilige Anteil der beiden an der gemessenen "Spezifischen Herzleistung" ist - hat sich nur die Körperleistung geändert, oder auch die Herzleistung? Ebenso ist nachteilig, dass eine "Spezifische" Leistung und eine "Spezifische" Herzleistung, also leistungsäquivalente Größen bestimmt werden, weil deshalb ein Vergleich mit z. B. der PWC aus medizinischen Ergometermessungen schwierig ist. Als nachteilig wird ferner angesehen, dass das Verfahren nach DPMA 10 2010 020 752.7 erfordert, dass immer auf einer Strecke identischer Länge trainiert wird und Strecken unterschiedlicher Längen nicht direkt verglichen werden können. Grundsätzlich wird für nachteilig auch gehalten, dass nur auf Laufbändern und Fahrradergometern eine Messung oder Berechnung der tatsächlichen (physikalischen) Körperleistung beispielsweise in der Einheit W (Watt) möglich ist, nicht aber bei Training in freiem Gelände (außer bei Fahrrädern, s. u.).

Als Nachteil von Heimtrainingsgeräten wird angesehen, dass Parameter wie MET oder PWC oder eine Herzleistung nicht ermittelt werden.

Insgesamt ist als Nachteil festzustellen, dass derzeit kein Parameter existiert, der die Herzleistung beschreibt und ständig und kontinuierlich auch unter sportlicher Belastung (Training in freiem Gelände) gemessen werden kann. Insbesondere ist es darüber hinaus nicht möglich, eine Herzleistung als Funktion der Körperleistung, also bei unterschiedlichen Körperleistungen, zu messen oder zu bestimmen.

Aufgabe der Erfindung ist daher die Schaffung eines Verfahrens, mit dem die genannten Nachteile vermieden werden. Das Verfahren soll eine einfache und regelmäßig anwendbare Messung der Herzleistung (im Sinne einer Leistungsfähigkeit) in Form einer Herzarbeit (Körperarbeit je Herzschlag), einer Herzarbeitskapazität und einer Körperleistung und deren Veränderungen ermöglichen. Herzarbeit und Herzarbeitskapazität sollen bei Belastung sowohl auf dem Ergometer, als auch beim sportlichen Training kontinuierlich und ständig gemessen werden können. Herzarbeit und Herzarbeitskapazität sollen vor allem als Funktion der Körperleistung und auch der Herzfrequenz bestimmt werden. Die Körperleistung und die Herzfrequenz sollen dafür alle Werte annehmen, die die jeweilige Person erreichen kann, so dass ein vollständiges Bild der Herz- und Körperleistung erfasst wird. Die Leistung, oder die Arbeit sollen in physikalischen Leistungs- oder Arbeitseinheiten bestimmt werden. Wo das nicht möglich ist, sollen geeignete Leistungs- und Arbeitsäquivalente ermittelt werden. In verschiedenen Ausführungsformen soll das Verfahren an medizinischen Ergometern, Heimtrainingsgeräten und vor allem beim individuellen Ausdauertraining unterschiedlicher Art in freiem Gelände ständig verwendbar sein. Die ermittelte Herzleistung (Herzarbeit) und die ermittelte Körperleistung sollen unmittelbar und direkt Aufschluss darüber geben, ob und in welchem Maße das Herz und der Körper durch das Training leistungsfähiger geworden sind, oder durch fehlendes Training an Leistungsfähigkeit verloren haben. Das Verfahren soll universell einsatzbar sein, d. h. für unterschiedliche Sportarten, für Übungsstrecken unterschiedlicher Länge und Beschaffenheit, mit Messung der Streckenlänge und auch ohne diese, für unterschiedliche Berechnungsverfahren der Körperleistung oder eines Äquivalents dieser. Das Verfahren soll ferner von jedem Laien angewandt werden können. Schließlich soll seine Anwendung keine aufwändige Messtechnik erfordern, sondern mit im Breitensport, bzw. in der Labormedizin üblichen Messgeräten anzuwenden sein.

Zur Lösung der Aufgabe wird ein neues Verfahren zur Ermittlung von Herz- und Körperleistungsparametern entwickelt. Es wird vor allem bei jeder Form sportlicher Bewegung (Gehen, Laufen, Radfahren, inline-skating, Schilanglauf, Schwimmen, etc.) angewandt, kann außerdem auch in Ruhe zur Bestimmung der Ruheparameter von Herz- und Körperleistung (bzw. Herzarbeit) verwendet werden. Es basiert auf der physiologischen Wirkung regelmäßiger sportlicher Betätigung, die Herzfrequenz zu verringern, d. h. die gleiche Körperleistung wird nach längerem Training bei geringerer Herzfrequenz erreicht. Das gilt auch in der Ruhe, d. h. die Leistung zur Erhaltung der Körperfunktionen (gegeben durch den energetischen Grundumsatz) wird bei geringerer Herzfrequenz erbracht. Der Ruhepuls wird also geringer, wobei der Grundenergieumsatz der gleiche bleibt. Dazu verwendet das Verfahren zwei neue Parameter, die die Leistung (Leistungsfähigkeit) des Herzens beschreiben: eine Herzarbeit $E_H$ und eine davon abgeleitete Herzarbeitskapazität $E_{HK}$. Die Herzarbeit $E_H$ wird aus einer Körperleistung $P_K$ und einer Herzfrequenz $f_H$ als Körperarbeit je Herzschlag bestimmt. {*Unter Bezug auf obige Anmerkung zur P WC wird hier fortgeführt: die Herzarbeit $E_H$ hat die Einheit [Ws] ist also tatsächlich eine Arbeit/Energie, die Herzarbeitskapazität $E_{HK}$ hat die Einheit [Ws/kg], ist also auch eine Arbeit. Deshalb wird für letztere der Englische Begriff Cardiac Working Capacity CWC in Anlehnung an die PWC (CWC wird hier aber korrekt benutzt) gewählt.*} Die Bestimmung der Körperleistung $P_K$ bei sportlicher Bewegung ist, mit Ausnahme von Ergometern, in aller Regel schwierig. Deshalb werden hier verschiedene, meist erfinderische Verfahren verwendet, die eine Körperleistung $P_K$ auf verschiedene Arten ermitteln.

Medizinische Ergometer (Laufbänder und Fahrräder) ermitteln die Körperleistung $P_K$ mittels integrierter Messeinrichtungen, sie wird im erfinderischen Verfahren direkt verwendet.

Beim Gehen und Laufen in freiem Gelände wird die Körperleistung $P_K$ in Form einer Laufkörperleistung $P_{LKL}$ nach technisch bekannten Verfahren berechnet (z. B. nach Margaria, oder Schulz, oder Eschenbacher, o. a., siehe dazu: Sascha Härtel, Entwicklung und Analyse walkingbasierter Ausdauertestverfahren im Rahmen der medizinischen Rehabilitation, Karlsruher sportwissenschaftliche Beiträge Bd. 4, Universitätsverlag Karlsruhe, 2007, ISBN 978-3-86644-171-2.). Dazu wird beim Training ein Sportcomputer verwendet. Dieser misst und speichert die Parameter: Zeit t, Herzfrequenz $f_H$, zurückgelegte Strecke s, Geländeneigung i, Geschwindigkeit v. Außerdem wird das Körpergewicht m inklusive Kleidung und allem mitgeführten Gerät gemessen und gespeichert. Aus den Parametern wird die Laufkörperleistung $P_{LKL}$ berechnet.

Beim Fahrradfahren in freiem Gelände wird die Körperleistung $P_K$ indirekt gemessen. Am Fahrrad befindet sich dazu eine Vorrichtung zur Messung der vom Fahrer auf die Pedale übertragenen Leistung als Körperleistung $P_K$ des Fahrers. Der mitgeführte Sportcomputer misst die Herzfrequenz $f_H$, das Gewicht m von Fahrer plus Fahrrad werden gesondert gemessen.

Analog wird bei stationären Trainingsfahrrädern verfahren, außerdem bei allen Trainingsgeräten, die über eine Messung der Körperleistung $P_K$ verfügen (hier geht das Gewicht der Geräte natürlich nicht in die Berechnung ein).

Ist für eine Bewegungsart keine Messung oder Berechnung der Körperleistung $P_K$ möglich, wohl aber eine Bestimmung der Bewegungsgeschwindigkeit v, dann wird als Körperleistung $P_K$ in Form einer dritten Variante ein Körperleistungsäquivalent $P_{K\ddot{a}}$ ermittelt als Produkt aus Körpergewicht m und Geschwindigkeit v. Es kann bei jedem Training in freiem Gelände verwendet werden, welches mit einer Fortbewegung verbunden ist, wie Gehen, Laufen, Wandern, Radfahren, Inlineskaten, Schwimmen, Rudern, Schilanglauf, etc.. In dieser Ausführungsform des erfindungsgemäßen Verfahrens werden drei leistungsäquivalente Parameter bestimmt: neben dem Körperleistungsäquivalent $P_{K\ddot{a}}$, ein Herzarbeitsäquivalent $P_{H\ddot{a}}$ und ein Herzarbeitskapazitätsäquivalent $P_{HK\ddot{a}}$.

Eine umfassende Analyse der ermittelten Herz- und Körperleistungsparameter ist Bestandteil des Verfahrens. In allen Fällen werden die absolute und relative Zu- oder Abnahme (bewirkt durch Training oder Trainingsmangel) der jeweiligen Leistungs/arbeitsparameter und der leistungsäquivalenten Parameter durch Vergleich der Messergebnisse im Verlauf der Zeit T ermittelt. Dabei definiert T die Zeitpunkte aufeinanderfolgender Trainingseinheiten und wird zweckmäßig als Datum, bzw. bei mehr als einer Trainingseinheit an einem Tag als Datum und Uhrzeit des Trainingsbeginns, dargestellt. Insbesondere wird die zeitliche Änderung der Herzarbeit $E_H=f(T)$ und der Herzarbeitskapazität $E_{HK}=f(T)$ als Funktion der Körperleistung $P_K$, der Laufkörperleistung $P_{LKL}$, oder des Körperleistungsäquivalents $P_{K\ddot{a}}$ und auch als Funktion der Herzfrequenz $f_H$ ermittelt. So werden für jede Körperleistung $P_K$, jede Laufkörperleistung $P_{LKL}$, jedes Körperleistungsäquivalent $P_{K\ddot{a}}$ und jede Herzfrequenz $f_H$ der Stand und die zeitliche Entwicklung der Herzarbeit $E_H(T)$ und der Herzarbeitskapazität $E_{HK}(T)$ erkennbar. Ebenso wird die zeitliche Änderung der Herzfrequenz $f_H=f(T)$ als Funktion der Körperleistung $P_K$, oder der Laufkörperleistung $P_{LKL}$, oder des Körperleistungsäquivalents $P_{K\ddot{a}}$ und auch als Funktion der Herzarbeit $E_H$ und der Herzarbeitskapazität $E_{HK}$ bestimmt. Die Herzfrequenz $f_H(T)$ wird dabei als leistungsbezogene Herzfrequenz dargestellt, d. h. als die Frequenz, die bei einer bestimmten Leistung (Körperleistung $P_K$, oder Laufkörperleistung $P_{LKL}$, oder Körperleistungsäquivalent $P_{K\ddot{a}}$, oder Herzarbeit $E_H$ oder auch Herzarbeitskapazität $E_{HK}$) im Laufe der Zeit T auftritt. So werden für jede Körperleistung $P_K$, oder jede Laufkörperleistung $P_{LKL}$, oder jedes Körperleistungsäquivalent $P_{K\ddot{a}}$ und für jede Herzarbeitskapazität $E_{HK}$ der Stand und die zeitliche Entwicklung der Herzfrequenz $f_H(T)$ erkennbar. Auch absolute und relative Unterschiede in der Veränderung aller Leistungsparameter werden durch Vergleich in technisch bekannter Weise bestimmt. Darüber hinaus wird von den erfindungsgemäßen Leistungsparametern ein Bezug zu (bzw. eine Umrechnung zu) medizinisch etablierten Parametern wie PWC und MET hergestellt, wodurch ein Vergleich mit den etablierten Verfahren und Messergebnissen möglich ist.

Nachfolgend werden die im Verfahren ermittelten Messparameter und die verwendete Messtechnik vorgestellt. Die Berechnung der Leistungsparameter und die Ergebnisanalyse werden beschrieben. Dabei wird die Anwendung des Verfahrens beispielhaft für alle Ausdauertrainingsarten in freiem Gelände, auch speziell für das Gehen/Laufen in freiem Gelände, sowie für stationäre Trainingsgeräte und für medizinische Ergometer erklärt. Auch die Anwendungen sowohl mit als auch ohne Wegmessung bzw. Geschwindigkeitsmessung werden beschrieben, ebenso wie die in Ruhe.

Erfindungsgemäß wird zur Beschreibung der Herzleistung eine Herzarbeit $E_H(j)$ ermittelt, als die vom Körper bei einem Herzschlag S (innerhalb der Zeitdauer eines Herzschlags S) umgesetzte Arbeit E (Energie). Die Herzarbeit $E_H(j)$ wird alternativ und physikalisch exakt auch "Körperarbeit je Herzschlag" genannt. Sie wird als mittlere Herzarbeit $E_H(j)$ für ein Zeitintervall $\Delta t$ nach Gleichung (1) aus der mittleren Körperleistung $P_K(j)$ in dem Zeitintervall $\Delta t$ und der mittleren Herzfrequenz $f_H(j)$ im Intervall $\Delta t$ berechnet. Der Index j bedeutet, dass die Körperleistung beliebige unterschiedliche Werte in der Spanne der möglichen Körperleistungen einer Person annehmen kann. Die Größe der Körperleistung $P_K(j)$ hängt ab von der Intensität der körperlichen Belastung (Bewegungsintensität, Trainingsintensität) und wird über diese variiert und eingestellt, so dass alle möglichen Körperleistungen $P_K(j)$ bei der Messung erfasst werden können.

Alternativ kann die Herzarbeit $E_H(j)$ für ein Zeitintervall $\Delta t$ auch als Momentanwert nach Gleichung (1) aus der Körperleistung $P_K(j)$ am Ende des Zeitintervalls $\Delta t$ und der Herzfrequenz $f_H(j)$ am Ende des Intervalls $\Delta t$ berechnet werden, es werden dann also nicht Mittelwerte bestimmt. Das Zeitintervall $\Delta t$ hat in beiden Fällen beispielsweise die Dauer von einigen Sekunden (1, 2, 3, 5, 10, 15, etc., o. ä.) oder Minuten (1, 2, 3, 5, 10, 15, etc., o. ä), bei langen Trainingseinheiten (z. B. Marathonlauf, etc.) auch einigen 10 Minuten. Bevorzugt wird eine Zeitdauer für $\Delta t$ von 2 bis 3 Minuten, das ist die Zeit, in der sich die Herzfrequenz $f_H$ einer Belastungsänderung (Änderung der Körperleistung $P_K$) anpasst. Der/die Momentanwerte können auch zu einem beliebigen Zeitpunkt bestimmt werden. Die Ermittlung der Körperleistung $P_K$ und ihrer Varianten wird später erläutert.

$$E_H(j)= P_K(j)/f_H(j) \qquad\qquad (1)$$

mit:

$E_H$: Herzarbeit [Ws/S], oder Körperarbeit in Ws je Herzschlag S
$P_K$: Körperleistung [W]
$f_H$: Herzfrequenz in Anzahl S der Schläge pro Sekunde s [S/s].

Da bei regelmäßigem Training die Herzfrequenz $f_H$ sinkt, nach Gl. (1) die Herzarbeit $E_H$ mit sinkender Herzfrequenz $f_H$ steigt und umgekehrt, ist die erfinderische Herzarbeit $E_H$ ein direktes Maß für die Körper- und besonders die Herzleistungsfähigkeit (Fitness).

Die Herzfrequenz $f_H(j)$ kann auch in Anzahl S der Schläge pro Minute (die übliche Einheit des "Puls") angegeben werden, entsprechend ändern sich alle davon betroffenen Einheiten.

Erfindungsgemäß wird weiter eine Herzarbeitskapazität $E_{HK}(j)$ ermittelt, als eine Herzarbeit $E_H(j)$ pro Kilogramm Körpergewicht m. Physikalisch handelt es sich um die Körperarbeit, die pro kg Körpergewicht mit einem Herzschlag S (innerhalb der Zeitdauer eines Herzschlags S) erbracht wird. Sie ermöglicht den Vergleich bei Menschen unterschiedlichen Gewichts und wird für jede Körperleistung $P_K(j)$ nach Gleichung (2) berechnet.

$$E_{HK}(j) = E_H(j) / m \qquad\qquad (2)$$

mit:

$E_{HK}$: Herzarbeitskapazität [W s/(S kg)]
m: Körpergewicht [kg] .

Die Herzarbeitskapazität wird alternativ auch als "Körperarbeitskapazität je Herzschlag" bezeichnet. Das Verfahren ermittelt somit für jede Körperleistung $P_K(j)$ die zugehörige Herzfrequenz $f_H(j)$, die Herzarbeit $E_H(j)$ und die Herzarbeitskapazität $E_{HK}(j)$ (jeweils als Mittelwerte in einem Zeitintervall $\Delta t$, oder als Momentanwerte zu einem beliebigen Zeitpunkt).

Ziel der Erfindung ist es, die physiologischen Herz- und Körperleistungsparameter zu einem beliebigen Zeitpunkt zu bestimmen und vor allem ihre Veränderungen im Laufe der Zeit und bei regelmäßigem Ausdauertraining oder sportlichem Training durch regelmäßig wiederholte Messungen zu ermitteln. Das dazu angewandte Verfahren wird nachfolgend beschrieben.

Die zu untersuchende Person führt im Verlaufe einer Zeit T (wie ausgeführt entspricht T einem Datum) ein Training mit wiederholter sportlicher Bewegung (Gehen, Laufen, Radfahren, Schwimmen, etc.) zur Zeit T (explizit: zu den aufeinanderfolgenden Zeitpunkten $T_1$, $T_2$, $T_3$, ...... im Verlaufe der Zeit T) aus. Vor jedem Training zur Zeit T (zu den Zeitpunkten (bzw. zum Datum) $T_1$, $T_2$, $T_3$, ... im Verlaufe der Zeit T) wird das Körpergewicht m(T) der Person gemessen. Bei jedem Training/jeder sportlichen Bewegung zur Zeit T (zu den Zeitpunkten $T_1$, $T_2$, $T_3$, ... im Verlaufe der Zeit T) werden im Verlaufe einer Zeit t mehrfach in Zeitintervallen $\Delta t$ lückenlos aufeinanderfolgend (explizit: zu den Messzeitpunkten $t_1$, $t_2$, $t_3$, $t_4$, .... im Verlaufe der Zeit t) und zeitgleich die Herzfrequenz $f_H(T,t)$ und die Bewegungsgeschwindigkeit v(T,t) der Person, sowie eine Geländeneigung i(T,t) und die Zeit t(T) gemessen. Die gesamte Zeit t ist die Gesamtdauer des jeweiligen Trainings zur Zeit T und die Messzeitpunkte $t_1$, $t_2$, $t_3$, $t_4$, .... folgen innerhalb der Zeit t in zeitlichen Abständen von $\Delta t$ aufeinander. Jeweils zeitgleich mit den Messungen der Herzfrequenz $f_H(T,t)$ und der Bewegungsgeschwindigkeit v(T,t), sowie der Geländeneigung i(T,t) wird die Körperleistung $P_K(T,t)$ der Person ermittelt. Daraus wird die Herzarbeit $E_H(T,t)$ der Person nach Gleichung (1) bestimmt, indem die Körperleistung $P_K(T,t)$ durch die Herzfrequenz $f_H(T,t)$ geteilt wird. Es wird die Herzarbeitskapazität $E_{HK}(T,t)$ der Person nach Gleichung (2) ermittelt, indem die Herzarbeit $E_H(T,t)$ durch das Körpergewicht m(T) geteilt wird. Alle Messungen und ermittelten Parameter werden mit Datum (entspricht zweckmäßig T) und Uhrzeit (entspricht zweckmäßig t) markiert und gespeichert. Die Person passt bei jedem Training die Intensität des Trainings an ihre jeweilige Leistungsfähigkeit (Trainingsstand und Tagesform) derart an, dass möglichst alle ihr möglichen Leistungsstufen durchlaufen werden. Dabei beginnt das Training aus der Ruhe mit niedriger Intensität, langsam wird diese kontinuierlich oder in moderaten Stufen gesteigert bis zur höchsten. Jede Stufe wird mindestens für die Zeit $\Delta t$ beibehalten. Vorzugsweise beträgt $\Delta t$ zwei bis drei Minuten und jede Stufe wird vorzugsweise für die Zeit von zwei Mal $\Delta t$ beibehalten; im zweiten Intervall $\Delta t$ hat sich die Herzfrequenz $f_H$ an die geänderte Belastung angepasst. Auch die höchste Intensität wird eine Zeit lang gehalten (mindestens für die Zeit von $\Delta t$ oder zwei Mal $\Delta t$, s.o.), dann wird die Intensität wieder langsam reduziert (kontinuierlich oder in Stufen von mindestens der Zeit $\Delta t$ oder zwei Mal $\Delta t$, s.o.) bis zur Ruhe. Ein Training von 44 Minuten Dauer kann so beispielsweise 11 Stufen von jeweils 4 Minuten Dauer beinhalten, 6 Stufen in der Leistung ansteigend, danach 5 Stufen wieder absteigend, $\Delta t$ wäre dabei zwei Minuten und es lägen dann Werte aus 22 Messungen vor. Die Variation der Intensität wird durch Variation der Bewegungsgeschwindigkeit v(T,t) erreicht, von langsam nach schnell und wieder zurück. Auf diese Weise werden die Daten über eine längere Trainingszeit T (zu vielen aufeinanderfolgenden Zeitpunkten $T_1$, $T_2$, $T_3$, ... im Verlaufe der Zeit T) ermittelt und gespeichert. Wie weiter oben dargestellt, werden auch hier die Messwerte als Mittelwerte im Zeitintervall $\Delta t$ gemessen, oder alternativ als Momentanwerte am Ende des Intervalls $\Delta t$ (zu den Messzeitpunkten $t_1$, $t_2$, $t_3$, $t_4$, .... im Verlaufe der Zeit t). Die Bestimmung der Mittelwerte geschieht dabei in technisch bekannter Weise. Es werden so alle der Person möglichen Werte der Körperleistung $P_K(j)$ als $P_K(T,t)$ und alle möglichen Werte der Herzfrequenz $f_H(j)$ als $f_H(T,t)$ beim Training wiederholt auftreten und erfasst. Im Training im Verlauf der Zeit T wird die erreichbare Körperleistung $P_K(T,t)$ bis zu einem Höchstwert steigen, auch die höchste erreichbare Herzfrequenz $f_H(T,t)$ wird zunehmen. Aus dem Trainingsverlauf resultiert eine entsprechend große Anzahl von Werten der Herzarbeit $E_H(T,t)$ und der Herzarbeitskapazität $E_{HK}(T,t)$. Dabei ergeben sich die physiologischen Herz- und Körperleistungsparameter alternativ als Mittelwerte im Zeitintervall $\Delta t$, oder alternativ als Momentanwerte am Ende des Intervalls $\Delta t$, je nachdem ob die verwendeten Messwerte als Mittelwerte im Zeitintervall $\Delta t$ gemessen wurden, oder als Momentanwerte am Ende des Intervalls $\Delta t$. Aus der Summe der Messdaten wird die Entwicklung der physiologischen Herz- und Körperleistungsparameter in nachfolgend beschriebener Weise ermittelt.

Die zur Zeit T (zu den Zeitpunkten $T_1$, $T_2$, $T_3$, ... im Verlaufe der Zeit T und dabei zu den Messzeitpunkten $t_1$, $t_2$, $t_3$, $t_4$, .... im Verlaufe der Zeit t) erfassten Werte der Körperleistung $P_K(T,t)$ (vorliegend als Mittelwerte im Zeitintervall $\Delta t$, oder als Momentanwerte am Ende des Intervalls $\Delta t$) unterscheiden sich in der Größe je nach Trainingsintensität mehr oder weniger stark. Bei fast gleicher Intensität sind die Unterschiede sehr gering. Bei genau gleicher Intensität ergeben sich aufgrund der verwendeten Mess- und Rechenverfahren zumindest unterschiedliche Nachkommastellen. Es werden also kaum zwei ermittelte Werte der Körperleistung $P_K(T,t)$ identisch sein, aber jeder Wert wird sehr nahe liegende Nachbarwerte haben, die aus gleicher oder fast gleicher Trainingsintensität im Verlauf des gesamten Trainings herrühren. Eine getrennte Betrachtung dieser nahe beieinander liegenden Werte ist nicht sinnvoll, zweckmäßiger ist es, sie zu einem Wert zusammenzufassen. Dazu wird eine alle Werte der Körperleistung $P_K(T,t)$ umfassende Anzahl n gleich großer/breiter Intervalle der Körperleistung $\Delta P_K(n)$ gebildet. Aus allen, jedem einzelnen Intervall $\Delta P_K(n)$ innerhalb der Zeit t eines jeden Trainings zur Zeit T zugehörigen Werten der Herzarbeit $E_H(T,t,n)$ (vorliegend als Mittelwerte im Zeitintervall $\Delta t$, oder als Momentanwerte am Ende des Intervalls $\Delta t$) wird ein Mittelwert $E_{Hm}(T,n)$ gebildet, und jeder Mittelwert $E_{Hm}(T,n)$ wird dem zugehörigen Intervall $\Delta P_K(n)$ zugeordnet. Es werden also die gleichen und fast gleichen Werte eines Trainings zur Zeit T gemittelt. Für jeden der n Intervallwerte der Körperleistung $\Delta P_K(n)$ wird die Herzarbeit $E_{Hm}(T,n)$ im Verlauf der Zeit T als Funktion $E_{Hm}(\Delta P_K(n),T,n)=f(T)$ ermittelt (der Verlauf der Mittelwerte jedes Trainings der Zeit T als Funktion der Zeit T).

Ebenso wird aus allen, jedem einzelnen Intervall $\Delta P_K(n)$ innerhalb der Zeit t eines jeden Trainings zugehörigen Werten der Herzarbeitskapazität $E_{HK}(T,t,n)$ (vorliegend als Mittelwerte im Zeitintervall $\Delta t$, oder als Momentanwerte am Ende des Intervalls Δt) ein Mittelwert $E_{HKm}(T,n)$ gebildet, und jeder Mittelwert $E_{HKm}(T,n)$ wird dem zugehörigen Intervall $\Delta P_K(n)$ zugeordnet. Es werden also wieder die gleichen und fast gleichen Werte eines Trainings zur Zeit T gemittelt. Für jeden der n Intervallwerte der Körperleistung $\Delta P_K(n)$ wird die Herzarbeitskapazität $E_{HKm}(T,n)$ im Verlauf der Zeit T als Funktion $E_{HKm}(\Delta P_K(n),T,n)=f(T)$ ermittelt (der Verlauf der Mittelwerte jedes Trainings der Zeit T als Funktion der Zeit T).

Auf analoge Weise wird die Entwicklung der physiologischen Leistungsparameter bei allen auftretenden Herzfrequenzen $f_H(T,t)$ ermittelt. Dazu wird eine alle Werte der Herzfrequenz $f_H(T,t)$ umfassende Anzahl p gleich großer Intervalle $\Delta f_H(p)$ gebildet, und aus allen, jedem einzelnen Intervall $\Delta f_H(p)$ innerhalb der Zeit t zugehörigen Werten der Herzarbeit $E_H(T,t,p)$ (vorliegend als Mittelwerte im Zeitintervall Δt, oder als Momentanwerte am Ende des Intervalls Δt) wird ein Mittelwert $E_{Hm}(T,p)$ gebildet. Es werden also auch hier die gleichen und fast gleichen Werte eines Trainings zur Zeit T gemittelt. Jeder Mittelwert $E_{Hm}(T,p)$ wird dem zugehörigen Intervall $\Delta f_H(p)$ zugeordnet, und für jeden der p Intervallwerte der Herzfrequenz $\Delta f_H(p)$ wird die Herzarbeit $E_{Hm}(T,p)$ im Verlauf der Zeit T als Funktion $E_{Hm}(\Delta f_H(p),T,p)=f(T)$ ermittelt (der Verlauf der Mittelwerte jedes Trainings der Zeit T als Funktion der Zeit T).

Ebenso wird aus allen, jedem einzelnen Intervall $\Delta f_H(p)$ innerhalb der Zeit t zugehörigen Werten der Herzarbeitskapazität $E_{HK}(T,t,p)$ (vorliegend als Mittelwerte im Zeitintervall Δt, oder als Momentanwerte am Ende des Intervalls Δt) ein Mittelwert $E_{HKm}(T,p)$ gebildet, und die Mittelwerte $E_{HKm}(T,p)$ werden den zugehörigen Intervallen $\Delta f_H(p)$ zugeordnet, und für jeden der p Intervallwerte der Herzfrequenz $\Delta f_H(p)$ wird die Herzarbeitskapazität $E_{HKm}(T,p)$ im Verlauf der Zeit T als Funktion $E_{HKm}(\Delta f_H(p),T,p)=f(T)$ ermittelt.

Aus den Verläufen der physiologischen Leistungsparameter über die Zeit T wird deren prozentuale Veränderung (bezogen auf den Anfangswert oder Mittelwert aller) über die Zeit T bestimmt. Dazu werden für jedes Intervall der Körperleistung $\Delta P_K(n)$ die prozentuale Änderung $E_{Hp\ddot{A}}(T,n)$ der Herzarbeit $E_{Hm}(T,n)$ und die prozentuale Änderung $E_{HKp\ddot{A}}(T,n)$ der Herzarbeitskapazität $E_{HKm}(T,n)$, bezogen auf den Mittelwert $E_{Hm}(n)$ aller Werte der Herzarbeit $E_{Hm}(T,n)$ als $E_{Hp\ddot{A}m}(T,n)$ und den Mittelwert $E_{HKm}(n)$ aller Werte der Herzarbeitskapazität $E_{HKm}(T,n)$ als $E_{HKp\ddot{A}m}(T)$, und außerdem bezogen auf den Anfangswert $E_{Hm}(I,n)$ von $E_{Hnm}(T,n)$ als $E_{Hp\ddot{A}a}(T,n)$ und auf den Anfangswert $E_{HKm}(I,n)$ von $E_{HKm}(T,n)$ als $E_{Hp\ddot{A}a}(T,n)$ als Funktion der Zeit T in technisch bekannter Weise bestimmt.

Analog werden für jedes Intervall der Herzfrequenz $\Delta f_H(p)$ die prozentuale Änderung der Herzarbeit $E_{Hpm}(T,p)$ und der Herzarbeitskapazität $E_{HKpm}(T,p)$, bezogen auf den jeweiligen Mittelwert und bezogen auf den jeweiligen Anfangswert als Funktion der Zeit T bestimmt.

In den vorstehenden Fällen und allen entsprechenden nachfolgenden werden nicht nur die prozentualen, sondern auch die absoluten Änderungen der ermittelten Parameter in technisch bekannter Weise bestimmt.

Die Intervalle für die Körperleistung $\Delta P_K(n)$ haben beispielsweise die Breite 1, 2, 3, 4, 5, 10, o. ä. Watt, bzw. Wattäquivalent. Sie sind charakterisiert durch die Körperleistung $P_k$ an der unteren und oberen Intervallgrenze, also $P_{Ku}(n)$ und $P_{Ko}(n)$. Die Herzfrequenz $f_H(T,t)$ wird als Zahl der Herzschläge pro Sekunde oder pro Minute gemessen und die Intervalle für die Herzfrequenz $\Delta f_H(p)$ haben beispielsweise die Breite 1, 2, 3, 4, o. ä. Schläge pro Minute oder pro Sekunde. Die Intervalle sind charakterisiert durch die Herzfrequenz $f_H$ an der unteren und oberen Intervallgrenze, also $f_{Hu}(p)$ und $f_{Ho}(p)$.

Die Ermittlung der Körperleistung $P_K$ erfolgt für unterschiedliche Bewegungs/Trainingsarten auf unterschiedliche Arten, die nachfolgend erläutert werden.

Beim Gehen und Laufen im Gelände oder auf einem Laufband wird die Körperleistung $P_K(T,t)$ als eine Laufkörperleistung $P_{LKL}(T,t)$ aus dem Körpergewicht $m(T)$, der Bewegungsgeschwindigkeit $v(T,t)$ und der Geländeneigung/Laufbandneigung $i(T,t)$ unter Anwendung einer der drei folgenden physiologischen Bewegungsgleichungen, oder entsprechender anderer Gleichungen ermittelt (Quelle: Sascha Härtel, s. o.):

$$P_K(T,t) = P_{LKL}(T,t) = [(2,11+0,25*i(T,t))*v(T,t)*m(T)+2,2*m(T)-151]/10,5 \qquad (3)$$

oder

$$P_K(T,t) = P_{LKL}(T,t) = [1,065+0,0511*i(T,t)+9,322*10^{-4}*i^2(T,t)]*v(T,t)*m(T)/4 \qquad (4)$$

oder

$$P_K(T,t) = P_{LKL}(T,t) = \{1,75+[1,065+0,0511*i(T,t)+9,322*10^{-4}*i^2(T,t)]*v(T,t)\}*m(T) \qquad (5)$$

Wo es technisch möglich ist, z. B. auf einem Laufband, beim Fahrradfahren im Gelände, oder auf einem stationären Fahrrad, wird die Körperleistung $P_K(T,t)$ mittels Leistungsmesseinrichtungen am Laufband oder Fahrrad bestimmt.

Bei allen Arten der Bewegung, (mit oder ohne jedem geeigneten Gerät, wie z. B. Laufen, Radfahren, Schwimmen, Rudern, Inline-skating, Skilanglauf,), insbesondere aber, wenn eines der vorstehenden Verfahren nicht möglich ist, wird die Körperleistung $P_K(T,t)$ als ein Körperleistungsäquivalent $P_{Kä}(T,t)$ als Produkt aus Körpergewicht m und Bewegungsgeschwindigkeit v zu

$$P_{Kä}(T,t) = m(T) * v(T,t) \tag{6}$$

ermittelt. Außerdem werden ein Herzarbeitsäquivalent $E_{Hä}(T,t)$ und ein Herzarbeitskapazitätsäquivalent $E_{HKä}(T,t)$ unter Anwendung der Gleichungen (1) und (2) sowie (6) in analoger Weise ermittelt. Die Bestimmung der zeitlichen Verläufe der physiologischen Parameter erfolgt wie vorstehend beschrieben. Wird Gerätschaft (Boot, Schi und Stöcke, etc.) bei der Bewegung mitgeführt, so schließt das Körpergewicht m das Gewicht der Gerätschaft ein.

Eine weitere Anwendungsweise des Verfahrens wird verwendet, wenn nur die Parameter Körpergewicht m, Zeit t und Herzfrequenz $f_h$ gemessen werden, also der Weg s und die Geschwindigkeit v nicht gemessen werden. Sie ist bei allen Sportarten möglich, die mit einer Fortbewegung verbunden sind. Als Messgeräte werden einfachste Herzfrequenzmesser (Pulsuhren) mit Uhr und Stoppuhrfunktion benutzt. Die bei Fortbewegung vom Körper geleistete mechanische Arbeit (umgesetzte Energie) E ist proportional zur bewegten Masse m (Körpergewicht m) und zur zurückgelegten Strecke der Länge s, nachfolgend "Strecke s" genannt. Erfindungsgemäß wird zur Bestimmung der Körperleistung $P_K$ und der Herzleistung $P_H$ die Fortbewegung im sportlichen Training wiederholt über dieselbe Strecke s durchgeführt. Daher ist die Arbeit E immer dieselbe, unabhängig von der zum Zurücklegen der Strecke s benötigten Zeit dt. Die Länge der Strecke s muss daher nicht bekannt sein. Die bei Fortbewegung im sportlichen Training über eine gegebene Strecke s vom Körper erbrachte Körperleistung $P_K$ ist umgekehrt proportional zu der zum Zurücklegen der Strecke s benötigten Zeit dt und proportional zur Masse m. Es wird ein weiteres, modifiziertes Körperleistungsäquivalents $P_{Käl}$ ermittelt, als Quotient aus dem Körpergewicht m und der Zeit dt, die zum Zurücklegen der Strecke s benötigt wird. Gemessen wird dazu das jeweilige Körpergewicht m und die Zeit dt, die zum Zurücklegen der Strecke s benötigt wird. Im wiederholten Training im Verlaufe der Zeit T wird im Verlaufe der Zeit t die Strecke s mehrfach mit unterschiedlicher Geschwindigkeit (Leistung) analog vorstehender Erläuterungen durchmessen (Laufen, Radfahren, Rudern, Schilanglaufen, etc.) und die dazu jeweils benötigte Zeit dt(T,t) gemessen und aufgezeichnet. Die Länge der Strecke s wird nicht gemessen:

$$P_{Käl}(T,t) = m(T) / dt(T,t) \tag{7}$$

$P_{Käl}$: Körperleistung, als Körperleistungsäquivalent
m: Körpergewicht, Masse
dt: Zeit, die zum Zurücklegen der Strecke s benötigt wird.

Die Strecke s wird so ausgewählt, dass zu ihrem Zurücklegen auch bei hoher Fortbewegungsgeschwindigkeit eine Zeit dt von mindestens einigen Minuten (wenigstens zwei bis drei Minuten) benötigt wird. Das gesamte Training sollte jeweils mindestens etwa 30 Minuten betragen. Die Strecke s wird also mehrmals hintereinander durchlaufen, quasi in mehreren Runden. Zur Unterscheidung der Runden wird (T,t) in Datum und Uhrzeit angegeben und aufgezeichnet. Sowohl das gesamte Training, als auch die Zeit dt können alternativ auch viele Minuten und bis zu Stunden betragen, z. B. beim Marathonlauf, beim Langstreckenradfahren, oder beim Wandern. Zur Auswertung der Messungen werden ein Herzarbeitsäquivalent $E_{Häl}(T)$ und ein Herzarbeitskapazitätsäquivalent $E_{HKäl}(T)$ unter Anwendung der Gleichungen (1) und (2) sowie (7) in analoger Weise ermittelt (T,t jeweils als Datum mit Uhrzeit). Die Bestimmung der zeitlichen Verläufe f(T) der physiologischen Parameter erfolgt wie vorstehend beschrieben.

Alternativ werden aber auch von Strecke s sich unterscheidende Strecken $s_n$ (andere Wege) verwendet, sofern sie dieselbe Länge s haben und hinsichtlich ihrer physikalischen Eigenschaften/Beschaffenheit (Bodenbelag, Steigung, Gefälle) identisch sind. Es können auch mehrere Strecken $s_m$ benutzt werden, die unterschiedliche Länge und Beschaffenheit haben, wenn zur Anwendung des Verfahrens immer die Messwerte derselben Strecken verwendet werden.

Zur allgemeinen Vergleichbarkeit bei allen Anwendungen des Verfahrens werden alternativ Referenzstrecken zum Training und zur Messung verwendet, die eine bekannte Länge und gleiche Beschaffenheit haben. Beim Gehen, Joggen, Laufen wird z. B. die "Rundbahn" eines Leichtathletikstadions verwendet, eine genormte, ovale 400-Meter-Laufbahn mit definierten physikalischen Eigenschaften. Durch teilweises, einmaliges oder mehrmaliges Durchlaufen der Rundbahn werden so genormte, vergleichbare Strecken $s_i$ unterschiedlicher Länge verfügbar. Für andere Sportarten werden vergleichbare, von offiziellen Sportverbänden standardisierte Strecken verwendet (z. B. für das Rudern eine Strecke von 500 m, 1000 m, oder 2000 m, und weitere).

Regelmäßiges Ausdauertraining über die Zeit T führt auch zu einer geringeren Herzfrequenz in Ruhe $f_{HR}$ (Ruhepuls). Zur quantitativen Ermittlung dieses Effekts wird zunächst die Körperleistung $P_K$ in Ruhe als Körperruheleistung $P_{KR}(T)$ (in Watt) mittels bekannter Gleichungen berechnet, (z. B. Harris-Benedict-Formel zur Berechnung des Grundumsatzes, oder anderer) für Männer:

$$P_K(T) = P_{KR}(T) = 4186{,}8*(66 + 13.7 * m(T) + 5 * h - 6.8 * a(T))/86400 \qquad (8)$$

und für Frauen:

$$P_K(T) = P_{KR}(T) = 4186{,}8*(655 + 9.6 * m(T) + 1.8 * h - 4.7 * a(T))/86400 \qquad (9)$$

mit:

$P_{KR}(T)$: Körperleistung in Ruhe in Watt
$m(T)$ : Körpergewicht (unbekleidet) in kg
$h$ : Körpergröße in cm
$a(T)$ : Alter in Jahren
Ausdruck in der Klammer: Grundumsatz je Tag in kcal.

Alternativ wird die Broca-Index Anpassung oder die Mifflin-St.Jeor-Formel, o. ä. verwendet (Harris J, Benedict F: A Biometric Study of Human Basal Metabolism. In: Proc Sei U S a. 4, Nr. 12, 1918, S. 370-3. doi:10.1073/pnas.4.12.370. PMID 16576330. Volltext bei PMC: 1091498. Mifflin, St Jeor et al: A new predictive equation for resting energy expenditure in healthy individuals. In: American Journal of Clinical Nutrition. 51, Nr. 2, Comparison of Predictive Equations for Resting Metabolic Rate in Healthy Nonobese and Obese Adults: A Systematic Review, S. 241-247. PMID 2305711).

Es wird dazu die Herzfrequenz $f_H(T)$ in Ruhe als Herzruhefrequenz $f_{HR}(T)$ mit einer Pulsuhr nach medizinisch üblichen Verfahren (ausgeruht, bewegungslos liegend, schweigend, mindestens zwei Minuten Ruhe vor der Messung, am besten immer zur gleichen Uhrzeit) einmal zu jeder Trainingszeit T (Datum) gemessen. Herzruhearbeit $E_{HR}(T)$ und Herzruhearbeitskapazität $E_{HRK}(T)$ werden aus der Körperruheleistung $P_{KR}(T)$ und der Herzruhefrequenz $f_{HR}(T)$ unter Anwendung der Gleichungen (1, 2) sowie (8, 9) ermittelt: $E_{HR}(T) = P_{KR}(T)/f_{HR}(T)$ und $E_{HRK}(T) = E_{HR}(T)/m(T)$. Aus allen Messungen der Zeit T werden der Verlauf, sowie die prozentualen und die absoluten Änderungen von Körperruheleistung $P_{KR}(T)$, Herzruhearbeit $E_{HR}(T)$ und Herzruhearbeitskapazität $E_{HRK}(T)$ über der Zeit T bestimmt.

Die Ermittlung der "Physical Working Capacity PWC" erfolgt, indem die mittels einer der Gleichungen (3, 4, 5, 8 oder 9) aus Messparametern, oder mittels Messeinrichtungen am Ergometer oder Fahrrad (wie vorstehend beschrieben) bestimmte Körperleistung $P_K$ durch das aktuelle Körpergewicht m dividiert wird:

$$PWC(T,t) = P_K(T,t)/m(T) \qquad (10)$$

Erfindungsgemäß wird das bekannte Verfahren zur Bestimmung der PWC dadurch erweitert, dass im Verlaufe des Trainings über die Zeit T die Physical Woprking Capacity $PWC(T,t)$ für jede auftretende Herzfrequenz $f_H(T,t)$ bestimmt wird und nicht nur bei den drei Herzfrequenzen 130, 150 und 170 Schläge pro Minute.

Analog den Auswertungen der zeitlichen Verläufe der Herzarbeit $E_H(T,t)$ und der Herzarbeitskapazität $E_{HK}(T,)$ wird auch für die Physical Working Capacity $PWC(T,t)$ der zeitliche Verlauf für jedes Herzfrequenzintervall $\Delta f_H(p)$ ermittelt. Dazu wird für jedes Training der Zeit T eine alle Werte der Herzfrequenz $f_H(T,t)$ umfassende Anzahl p gleich großer Intervalle $\Delta f_H(p)$ gebildet, und aus allen, jedem einzelnen Intervall $\Delta f_H(p)$ innerhalb der Zeit t zugehörigen Werten der Physical Working Capacity $PWC(T,t,p)$ (vorliegend als Mittelwerte im Zeitintervall $\Delta t$, oder als Momentanwerte am Ende des Intervalls $\Delta t$) wird ein Mittelwert $PWC_m(T,p)$ gebildet, und die Mittelwerte $PWC_m(T,p)$ werden den zugehörigen Intervallen $\Delta f_H(p)$ zugeordnet, und für jeden der p Intervallwerte der Herzfrequenz $\Delta f_H(p)$ wird die Physical Working Capacity $PWC(T,p)$ im Verlauf der Zeit T als Funktion $PWC_m(\Delta f_H(p),T,p)=f(T)$ ermittelt.

Analog werden für jedes Intervall der Herzfrequenz $\Delta f_H(p)$ die prozentuale Änderung der Physical Working

Capacity $PWC_{pm}(T,p)$, bezogen auf den jeweiligen Mittelwert aller Werte $PWC_{pm}(p)$ und bezogen auf den jeweiligen Anfangswert $PWC_{pm}(I,p)$ als Funktion der Zeit T bestimmt.

Die Umrechnung von PWC in Metabolic Equivalent of Task MET erfolgt in technisch bekannter Weise.

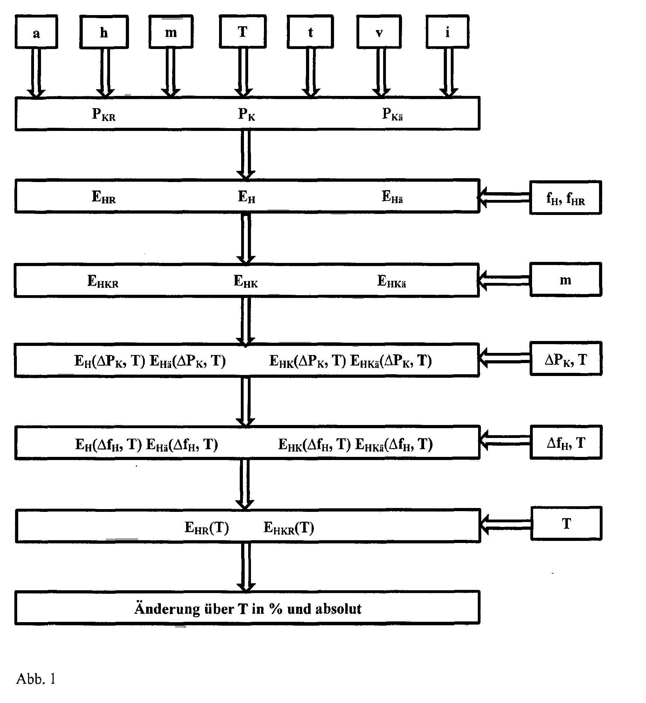

Zur Gesamtübersicht des Verfahrens zeigt Abbildung 1 ein Blockschaltbild aller Messgrößen/Eingabedaten und der daraus ermittelten physiologischen Parameter in einer funktionalen Struktur. Die oberen Blöcke der Abb. 1 stellen alle gemessenen Parameter dar: das Alter a der Person, die Körpergröße h sowie das Gewicht m, ferner die Zeit T (Datum), die Zeit t (Uhrzeit), die Bewegungsgeschwindigkeit v und die Geländeneigung i (die Strecke s ergibt sich aus der Geschwindigkeit v und der Zeit t). Im ersten Schritt wird die Körperleistung $P_K$ berechnet, je nach Messdaten in den verschiedenen Varianten $P_{KR}$ (in Ruhe), $P_K$ (im Training) und $P_{Kä}$ (als Leistungsäquivalent im Training). Im zweiten Schritt werden unter Verwendung der Herzfrequenz $f_H$ (im Training) oder $f_{HR}$ (in Ruhe) die verschiedenen Arten der Herzarbeit $E_H$ (im Training), $E_{HR}$ (in Ruhe) und $E_{Hä}$ (als Äquivalent im Training) bestimmt. Daraus erfolgt im dritten Schritt unter Verwendung des Körpergewichts m die Ermittlung der Varianten der Herzarbeitskapazität $E_{HKR}$, $E_{HK}$ und $E_{HKä}$. Darauf folgt die Bestimmung der zeitlichen Verläufe der Varianten von Herzarbeit $E_H$ und Herzarbeitskapazität $E_{HK}$ für alle n Intervalle der Körperleistung $\Delta P_K(n)$ und für alle p Intervalle der Herzfrequenz $\Delta f_H(p)$. Schließlich werden der Verlauf der Herzruhearbeit $E_{HR}$ und Herzruhearbeitskapazität $E_{HRK}$ über der Zeit T ermittelt. Zum Abschluss werden die prozentualen und absoluten Änderungen aller physiologischen Parameter als Funktion der Zeit T berechnet.

Als Messeinrichtung bei sportlicher Bewegung/Training im Freien wird ein tragbarer Sportcomputer verwendet, der über Vorrichtungen zur Ermittlung, Speicherung und Übertragung der Herzfrequenz $f_H$, der Geschwindigkeit v, der Geländeneigung i, der Zeit t und der Strecke s (die sich auch aus Geschwindigkeit v und Zeit t ergibt) verfügt. Dabei ist die Messung der Geschwindigkeit v und der Geländeneigung i mit unterschiedlichen, technisch bekannten Verfahren möglich, z. B. auch mit einer integrierten Einrichtung für die Satellitennavigation. Die Geländeneigung i wird meist nicht direkt gemessen, sondern aus der Messung des zurückgelegten Wegs und der Geländehöhendifferenz (Messung über GPS, oder barometrische Höhenmessung) berechnet. Für die Anwendung des Verfahrens ohne Weg- und ohne Geschwindigkeitsmessung kann als Messeinrichtung eine Pulsuhr (tragbarer Herzfrequenzmesser) mit Stoppuhrfunktion verwendet werden. Bei sportlicher Bewegung/Training in Räumen wird ein stationäres Sportgerät verwendet, das über Vorrichtungen zur Ermittlung, Speicherung und Übertragung der Herzfrequenz $f_H$, der Körperleistung $P_K$ und der Zeit t verfügt.

Bei allen Ausführungsformen des Verfahrens werden die Daten in technisch bekannter Weise aufgezeichnet und rechnerisch verarbeitet (Berechnung aller Parameter, wie CWC, etc.), wozu im Freien vorzugsweise der tragbare Sportcomputer verwendet. Bei sportlicher Bewegung/Training in Räumen verfügt vorzugsweise das stationäre Sportgerät über Vorrichtungen zur rechnerischen Verarbeitung (Berechnung aller Parameter, wie CWC, etc.). Alternativ wird für die Berechnungen aller Parameter, wie CWC, etc. ein Computer (PC, Notebook, etc.) verwendet, zu dem die gemessenen Daten in technisch bekannter Weise übertragen werden wird. Es können auch Mobiltelefongeräte verwendet werden, die sowohl über Einrichtungen zum Empfang von Daten der Pulsuhr oder des Sportcomputers, als auch zur Berechnung der erfindungsgemäßen Leistungsparameter, sowie deren Speicherung und Anzeige verfügen. Vom Mobiltelefon können alle Daten und Parameter in technisch bekannter Weise auch auf einen Computer oder Server übertragen werden und umgekehrt.

Das Verfahren kann auch bei Tieren, z. B. Rennpferden, Rennkamelen, Schlittenhunden, etc. angewandt werden. Die Messungen am Tier und beim Training werden mit geeigneten, technisch bekannten Messeinrichtungen vorgenommen. Die Auswertung findet wie vorstehend beschrieben statt.

**Patentansprüche**

1. Verfahren zur Bestimmung von physiologischen Herz- und Körperleistungsparametern, wobei:

   - eine Person im Verlaufe einer Zeit T wiederholt sportliche Bewegung (Gehen, Laufen, Radfahren, Schwimmen, etc.) ausführt und dabei
   - jedes Mal ein Körpergewicht m(T) der Person gemessen wird,
   - während jeder sportlichen Bewegung in der Zeit T im Verlaufe einer Zeit t mehrfach aufeinanderfolgend und zeitgleich eine Herzfrequenz $f_H(T,t)$ und eine Bewegungsgeschwindigkeit v(T,t) der Person, sowie eine Geländeneigung i(T,t) und die Zeit t(T) gemessen werden und eine Körperleistung $P_K(T,t)$ der Person ermittelt werden,
   - für jede ermittelte Körperleistung $P_K(T,t)$ eine Herzarbeit $E_H(T,t)$ als Körperarbeit je Herzschlag der Person bestimmt wird, indem die Körperleistung $P_K(T,t)$ durch die Herzfrequenz $f_H(T,t)$ geteilt wird,
   - für jede ermittelte Herzarbeit $E_H(T,t)$ eine Herzarbeitskapazität $E_{HK}(T,t)$ als Körperarbeitskapazität je Herzschlag der Person ermittelt wird, indem die Herzarbeit $E_H(T,t)$ durch das Körpergewicht m(T) geteilt wird,
   - eine alle Werte der Körperleistung $P_K(T,t)$ umfassende Anzahl n gleich großer Intervalle $\Delta P_K(n)$ gebildet wird, und aus allen, jedem einzelnen Intervall $\Delta P_K(n)$ innerhalb der Zeit t zugehörigen Werten der Herzarbeit $E_H$

(T,t,n) ein Mittelwert $E_{Hm}(T,n)$ gebildet wird, und die Mittelwerte $E_{Hm}(T,n)$ den zugehörigen Intervallen $\Delta P_K(n)$ zugeordnet werden, für jeden der n Intervallwerte der Körperleistung $\Delta P_K(n)$ die Herzarbeit $E_{Hm}(T,n)$ im Verlauf der Zeit T als Funktion $E_{Hm}(\Delta P_K(n),T,n)=f(T)$ ermittelt wird,

- aus allen, jedem einzelnen Intervall $\Delta P_K(n)$ innerhalb der Zeit t zugehörigen Werten der Herzarbeitskapazität $E_{HK}(T,t,n)$ ein Mittelwert $E_{HKm}(T,n)$ gebildet wird, und die Mittelwerte $E_{HKm}(T,n)$ den zugehörigen Intervallen $\Delta P_K(n)$ zugeordnet werden, und für jeden der n Intervallwerte der Körperleistung $\Delta P_K(n)$ die Herzarbeitskapazität $E_{HKm}(T,n)$ im Verlauf der Zeit T als Funktion $E_{HKm}(\Delta P_K(n),T,n)=f(T)$ ermittelt wird,

- eine alle Werte der Herzfrequenz $f_H(T,t)$ umfassende Anzahl p gleich großer Intervalle $\Delta f_H(p)$ gebildet wird, und aus allen, jedem einzelnen Intervall $\Delta f_H(p)$ innerhalb der Zeit t zugehörigen Werten der Herzarbeit $E_H(T,t,p)$ ein Mittelwert $E_{Hm}(T,p)$ gebildet wird, und die Mittelwerte $E_{Hm}(T,p)$ den zugehörigen Intervallen $\Delta f_H(p)$ zugeordnet werden, und für jeden der p Intervallwerte der Herzfrequenz $\Delta f_H(p)$ die Herzarbeit $E_{Hm}(T,p)$ im Verlauf der Zeit T als Funktion $E_{Hm}(\Delta f_H(p),T,p)=f(T)$ ermittelt wird,

- aus allen, jedem einzelnen Intervall $\Delta f_H(p)$ innerhalb der Zeit t zugehörigen Werten der Herzarbeitskapazität $E_{HK}(T,t,p)$ ein Mittelwert $E_{HKm}(T,p)$ gebildet wird, und die Mittelwerte $E_{HKm}(T,p)$ den zugehörigen Intervallen $\Delta f_H(p)$ zugeordnet werden, und für jeden der p Intervallwerte der Herzfrequenz $f_H(p)$ die Herzarbeitskapazität $E_{HKm}(T,p)$ im Verlauf der Zeit T als Funktion $E_{HKm}(\Delta f_H(p),T,p)=f(T)$ ermittelt wird,

- und für jedes Intervall der Körperleistung $\Delta P_K(n)$ die prozentuale Änderung $E_{Hp\ddot{A}}(T,n)$ der Herzarbeit $E_{Hm}(T,n)$ und die prozentuale Änderung $E_{HKp\ddot{A}}(T,n)$ der Herzarbeitskapazität $E_{HKm}(T,n)$, bezogen auf den Mittelwert $E_{Hm}(n)$ aller Werte der Herzarbeit $E_{Hm}(T,n)$ als $E_{Hp\ddot{A}m}(T,n)$ und bezogen auf den Mittelwert $E_{HKm}(n)$ aller Werte der Herzarbeitskapazität $E_{HKm}(T,n)$ als $E_{HKp\ddot{A}m}(T,n)$, und außerdem bezogen auf den Anfangswert $E_{Hm}(I,n)$ der Herzarbeit $E_{Hm}(T,n)$ als $E_{Hp\ddot{A}a}(T,n)$ und auf den Anfangswert $E_{HKm}(I,n)$ der Herzarbeitskapazität $E_{HKm}(T,n)$ als $E_{HKp\ddot{A}a}(T,n)$ als Funktion der Zeit T bestimmt werden,

- und analog für jedes Intervall der Herzfrequenz $\Delta f_H(p)$ die prozentuale Änderung der Herzarbeit $E_{Hpm}(T,p)$ und der Herzarbeitskapazität $E_{HKpm}(T,p)$, bezogen auf den jeweiligen Mittelwert und bezogen auf den jeweiligen Anfangswert als Funktion der Zeit T bestimmt werden,

- und dass in jedem Fall auch die absoluten Änderungen der Parameter Herzarbeit und Herzarbeitskapazität als Funktion der Zeit T ermittelt werden.

**2.** Verfahren nach Anspruch 1, wobei:

- beim Gehen und Laufen im Gelände oder auf einem Laufband die Körperleistung $P_K(T,t)$ als eine Laufkörperleistung $P_{LKL}(T,t)$ aus dem Körpergewicht $m(T)$, der Bewegungsgeschwindigkeit $v(T,t)$ und der Geländeneigung $i(T,t)$ unter Anwendung einer physiologischen Bewegungsgleichung ermittelt wird, wie:

$$P_K(T,t) = P_{LKL}(T,t) = [(2,11+0,25*i(T,t))*v(T,t)*m(T)+2,2*m(T)-151]/10,5$$

oder

$$P_K(T,t) = P_{LKL}(T,t) = [1,065+0,0511*i(T,t)+9,322*10^{-4} * i^2(T,t)] * v(T,t)*m(T)/4$$

oder

$$P_K(T,t) = P_{LKL}(T,t) = \{1,75+[1,065+0,0511*i(T,t)+9,322*10^{-4} * i^2(T,t)]*v(T,t)\}*m(T)$$

- wo es technisch möglich ist, z. B. beim Fahrradfahren, oder auf einem stationären Fahrrad, die Körperleistung $P_K(T,t)$ mittels Leistungsmesseinrichtungen am Fahrrad bestimmt wird,

- bei allen Arten der Bewegung die Körperleistung $P_K(T,t)$ als ein Körperleistungsäquivalent $P_{K\ddot{a}}(T,t)$ als Produkt aus Körpergewicht m und Bewegungsgeschwindigkeit v zu $P_{K\ddot{a}}(T,t) = m(T) * v(T,t)$, sowie ein Herzarbeitsäquivalent $E_{H\ddot{a}}(T,t)$ und ein Herzarbeitskapazitätsäquivalent $E_{HK\ddot{a}}(T,t)$ ermittelt werden,

**3.** Verfahren nach Anspruch 1, wobei:

- im Verlaufe der Zeit T bei allen Arten der Bewegung wiederholt im Verlaufe der Zeit t immer eine Strecke s derselben Länge und Beschaffenheit zurückgelegt wird,
- die Zeit $dt(T,t)$, die zum Zurücklegen der Strecke s benötigt wird, gemessen wird,
- die mittlere Herzfrequenz $f_{Hm}(T,t)$ beim Zurücklegen der Strecke s ermittelt wird,
- die Körperleistung $P_K(T,t)$ als ein Körperleistungsäquivalent $P_{Käl}(T,t)$ als Quotient aus Körpergewicht $m(T)$ und Zeit $dt(T,t)$ zu $P_{Käl}(T,t) = m(T) / dt(T,t)$, sowie ein Herzarbeitsäquivalent $E_{Häl}(T,t)$ als $E_{Häl}(T,t) = P_{Käl}(T,t) / f_H(T,t)$ und ein Herzarbeitskapazitätsäquivalent $E_{HKäl}(T,t)$ als $E_{HKäl}(T,t) = E_{Häl}(T,t) / m(T)$ ermittelt werden.

**4.** Verfahren nach Anspruch 1, wobei:

- die Körperruheleistung $P_{KR}(T)$ für Männer als

$$P_K(T) = P_{KR}(T) = 0{,}0485*(66 + 13.7 * m(T) + 5 * h - 6.8 * a(T))$$

und für Frauen als

$$P_K(T) = P_{KR}(T) = 0{,}0485*(655 + 9.6 * m(T) + 1.8 * h - 4.7 * a(T))$$

bestimmt wird
- die Herzruhefrequenz $f_{HR}(T)$ zu jeder Trainingszeit T (Datum) gemessen wird
- die Herzruhearbeit $E_{HR}(T)$ und Herzruhearbeitskapazität $E_{HRK}(T)$ als
- $E_{HR}(T) = P_{KR}(T)/f_{HR}(T)$ und $E_{HRK}(T) = E_{HR}(T)/m(T)$ ermittelt wird und
- der Verlauf, sowie die prozentualen Änderungen, bezogen auf den Mittelwert aller sowie bezogen auf den Anfangswert und die absoluten Änderungen von Körperruheleistung $P_{KR}(T)$, Herzruhearbeit $E_{HR}(T)$ und Herzruhearbeitskapazität $E_{HRK}(T)$ über der Zeit T bestimmt werden.

**5.** Verfahren nach einem der Ansprüche 1, 2 und 3, wobei:

- die Bewegungsgeschwindigkeit $v(T,t)$, die Geländeneigung $i(T,t)$ und die Herzfrequenz $f_H(T,t)$ innerhalb der Zeit t über ein jeweils gleich großes Zeitintervall $\Delta t$ von Sekunden, Minuten oder Stunden gemittelt werden, und damit jeweils Mittelwerte der Körperleistung $P_K(T,t)$, der Herzarbeit $E_H(T,t)$, und der Herzarbeitskapazität $E_{HK}(T,t)$ über das Zeitintervall $\Delta t$ ermittelt werden.

**6.** Verfahren nach Anspruch 1, wobei:

- die Intervalle für die Körperleistung $P_K(n)$ die Breite 1, 2, 3, 4, 5, 10, o. ä. Watt, bzw. Wattäquivalent haben, und
- diese Intervalle durch die Körperleistung $P_k$ an der unteren $P_{Ku}(n)$ und oberen $P_{Ko}(n)$ Intervallgrenze definiert sind,
- die Herzfrequenz $f_H(T,t)$ als Zahl der Herzschläge pro Sekunde oder pro Minute gemessen wird und die Intervalle für die Herzfrequenz $f_H(p)$ die Breite 1, 2, 3, 4, o. ä. Schläge pro Sekunde oder pro Minute haben, und
- diese Intervalle durch die Herzfrequenz $f_H$ an der unteren $f_{Hu}(p)$ und oberen $f_{Ho}(p)$ Intervallgrenze definiert sind.

**7.** Verfahren nach einem der Ansprüche 1, 2 und 3, wobei

- zur Ermittlung der Körperleistung $P_K(T,t)$, der Herzarbeit $E_H(T,t)$ und der Herzarbeitskapazität $E_{HK}(T,t)$ und ihrer Veränderung während einer Zeit T von Tagen, Wochen, Monaten, Jahren ein regelmäßig ein mindestens drei Mal pro Woche wiederholtes Bewegungstraining (Gehen, Laufen, Radfahren, etc.) jeweils über eine Zeit t von einigen Minuten bis zu Stunden bei wechselnder Bewegungsgeschwindigkeit $v(T,t)$ durchgeführt wird.

**8.** Verfahren nach einem der Ansprüche 1, 2, 5 und 6, wobei:

- für jede auftretende Herzfrequenz $f_H(T,t)$ eine Physical Working Capacity $PWC(T,t)$ ermittelt wird, indem die zugehörige Körperleistung $P_K(T,t)$ durch das Körpergewicht $m(T)$ dividiert wird, und
- für die Physical Working Capacity $PWC(T,t)$ der zeitliche Verlauf für jedes Herzfrequenzintervall $\Delta f_H(p)$ ermittelt

wird, indem eine alle Werte der Herzfrequenz $f_H(T,t)$ umfassende Anzahl p gleich großer Intervalle $\Delta f_H(p)$ gebildet wird, und aus allen, jedem einzelnen Intervall $\Delta f_H(p)$ innerhalb der Zeit t zugehörigen Werten der Physical Working Capacity PWC(T,t,p) ein Mittelwert $PWC_m(T,p)$ gebildet wird, und die Mittelwerte $PWC_m(T,p)$ den zugehörigen Intervallen $\Delta f_H(p)$ zugeordnet werden, und für jeden der p Intervallwerte der Herzfrequenz $\Delta f_H(p)$ die Physical Working Capacity PWC(T,p) im Verlauf der Zeit T als Funktion $PWC_m(\Delta f_H(p),T,p)=f(T)$ ermittelt wird, und

- für jedes Intervall der Herzfrequenz $\Delta f_H(p)$ die prozentuale Änderung der Physical Working Capacity $PWC_{pm}(T,p)$, bezogen auf den jeweiligen Mittelwert aller Werte $PWC_{pm}(p)$ und bezogen auf den jeweiligen Anfangswert $PWC_{pm}(I,p)$, als Funktion der Zeit T bestimmt.

9. Verfahren nach einem der Ansprüche 1, 2, 3, 6 und 7, wobei:

- die sportliche Bewegung auf einer genormten Strecke stattfindet.

10. Vorrichtung in Form einer Einrichtung zur Erfassung und Verarbeitung physiologischer Parameter zur Durchführung eines Verfahrens nach einem der Ansprüche 1 bis 4, wobei:

- für sportliche Bewegung im Freien ein mitgeführter tragbarer Sportcomputer die Herzfrequenz $f_H$, die Geschwindigkeit v, die Geländeneigung i, die Zeit t und die Strecke s ermittelt und speichert, sowie daraus die Herzarbeitskapazität $E_{HK}(T,t)$ und deren Änderung berechnet, und die gemessenen Parameter an einen Computer zur Berechnung der Herzarbeitskapazität $E_{HK}(T,t)$ und zur weiteren Verarbeitung überträgt,
- für sportliche Bewegung in Räumen ein stationäres Sportgerät die Herzfrequenz $f_H$, und die Körperleistung $P_K$ ermittelt, speichert, sowie daraus die Herzarbeitskapazität $E_{HK}(T,t)$ und deren Änderung berechnet, und die gemessenen Parameter an einen Computer zur Berechnung der Herzarbeitskapazität $E_{HK}(T,t)$ und zur
- weiteren Verarbeitung überträgt.

| a | h | m | T | t | v | i |

$$P_{KR} \qquad\qquad P_K \qquad\qquad P_{Kä}$$

$$E_{HR} \qquad\qquad E_H \qquad\qquad E_{Hä} \qquad\qquad\qquad f_H, f_{HR}$$

$$E_{HKR} \qquad\qquad E_{HK} \qquad\qquad E_{HKä} \qquad\qquad\qquad m$$

$$E_H(\Delta P_K, T)\ E_{Hä}(\Delta P_K, T) \qquad E_{HK}(\Delta P_K, T)\ E_{HKä}(\Delta P_K, T) \qquad \Delta P_K, T$$

$$E_H(\Delta f_H, T)\ E_{Hä}(\Delta f_H, T) \qquad E_{HK}(\Delta f_H, T)\ E_{HKä}(\Delta f_H, T) \qquad \Delta f_H, T$$

$$E_{HR}(T) \qquad E_{HKR}(T) \qquad\qquad\qquad T$$

**Änderung über T in % und absolut**

Abb. 1

Europäisches
Patentamt

European
Patent Office

Office européen
des brevets

**EUROPÄISCHER RECHERCHENBERICHT**

Nummer der Anmeldung

EP 13 00 5724

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | DE 10 2010 020752 A1 (KREATIVE TECHNOLOGIE LWU UG [DE]) 17. November 2011 (2011-11-17) * das ganze Dokument * ----- | 1,10 | INV. A61B5/024 A61B5/029 A61B5/22 A63B22/02 |
| A | US 2007/287596 A1 (CASE CHARLES W JR [US] ET AL CASE JR CHARLES WHIPPLE [US] ET AL) 13. Dezember 2007 (2007-12-13) * Absätze [0059], [0077], [0153] - [0156] * ----- | 1,10 | A63B22/06 A61B5/11 A61B5/0205 |

RECHERCHIERTE
SACHGEBIETE (IPC)

A61B
A63B

Der vorliegende Recherchenbericht wurde für alle Patentansprüche erstellt

| Recherchenort | Abschlußdatum der Recherche | Prüfer |
|---|---|---|
| München | 10. April 2014 | Manschot, Jan |

KATEGORIE DER GENANNTEN DOKUMENTE

X : von besonderer Bedeutung allein betrachtet
Y : von besonderer Bedeutung in Verbindung mit einer
     anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
     nach dem Anmeldedatum veröffentlicht worden ist
D : in der Anmeldung angeführtes Dokument
L : aus anderen Gründen angeführtes Dokument
...................................................................
& : Mitglied der gleichen Patentfamilie, übereinstimmendes
     Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 13 00 5724

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2014

| Im Recherchenbericht angeführtes Patentdokument | Datum der Veröffentlichung | Mitglied(er) der Patentfamilie | | Datum der Veröffentlichung |
|---|---|---|---|---|
| DE 102010020752 A1 | 17-11-2011 | DE | 102010020752 A1 | 17-11-2011 |
| | | EP | 2401960 A2 | 04-01-2012 |
| US 2007287596 A1 | 13-12-2007 | BR | PI0519077 A2 | 23-12-2008 |
| | | CA | 2593507 A1 | 22-06-2006 |
| | | CA | 2717401 A1 | 22-06-2006 |
| | | CN | 101111743 A | 23-01-2008 |
| | | CN | 101711915 A | 26-05-2010 |
| | | CN | 102198316 A | 28-09-2011 |
| | | CN | 102198317 A | 28-09-2011 |
| | | CN | 102198318 A | 28-09-2011 |
| | | CN | 102198319 A | 28-09-2011 |
| | | CN | 102205176 A | 05-10-2011 |
| | | CN | 102225230 A | 26-10-2011 |
| | | CN | 102225231 A | 26-10-2011 |
| | | CN | 102225232 A | 26-10-2011 |
| | | CN | 102225233 A | 26-10-2011 |
| | | CN | 102225234 A | 26-10-2011 |
| | | CN | 102225235 A | 26-10-2011 |
| | | CN | 103083872 A | 08-05-2013 |
| | | EP | 1825220 A2 | 29-08-2007 |
| | | EP | 2333489 A1 | 15-06-2011 |
| | | EP | 2333490 A1 | 15-06-2011 |
| | | EP | 2333491 A1 | 15-06-2011 |
| | | EP | 2336726 A1 | 22-06-2011 |
| | | EP | 2336727 A1 | 22-06-2011 |
| | | EP | 2336728 A1 | 22-06-2011 |
| | | EP | 2357446 A2 | 17-08-2011 |
| | | EP | 2357447 A2 | 17-08-2011 |
| | | EP | 2357448 A2 | 17-08-2011 |
| | | EP | 2357449 A2 | 17-08-2011 |
| | | EP | 2357450 A2 | 17-08-2011 |
| | | JP | 5260604 B2 | 14-08-2013 |
| | | JP | 5268155 B2 | 21-08-2013 |
| | | JP | 5313977 B2 | 09-10-2013 |
| | | JP | 5436707 B2 | 05-03-2014 |
| | | JP | 2008524589 A | 10-07-2008 |
| | | JP | 2010075715 A | 08-04-2010 |
| | | JP | 2010253302 A | 11-11-2010 |
| | | JP | 2010253303 A | 11-11-2010 |
| | | JP | 2013099673 A | 23-05-2013 |
| | | JP | 2013126586 A | 27-06-2013 |
| | | JP | 2013138880 A | 18-07-2013 |
| | | US | 2006136173 A1 | 22-06-2006 |
| | | US | 2007287596 A1 | 13-12-2007 |
| | | US | 2009319230 A1 | 24-12-2009 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.**

EP 13 00 5724

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2014

| Im Recherchenbericht angeführtes Patentdokument | Datum der Veröffentlichung | Mitglied(er) der Patentfamilie | Datum der Veröffentlichung |
|---|---|---|---|
| | | US 2010210421 A1 | 19-08-2010 |
| | | US 2012078396 A1 | 29-03-2012 |
| | | WO 2006065679 A2 | 22-06-2006 |
| | | ZA 200704967 A | 25-09-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Entwicklung und Analyse walkingbasierter Ausdauertestverfahren im Rahmen der medizinischen Rehabilitation. **SASCHA HÄRTEL.** Karlsruher sportwissenschaftliche Beiträge. Universitätsverlag Karlsruhe, 2007, vol. 4
- **SASCHA HÄRTEL.** Entwicklung und Analyse walkingbasierter Ausdauertestverfahren im Rahmen der medizinischen Rehabilitation. *Karlsruher sportwissenschaftliche Beiträge,* 2007, vol. 4, ISBN 978-3-86644-171-2

- **HARRIS J ; BENEDICT F.** A Biometric Study of Human Basal Metabolism. *Proc Sei U S a.,* 1918, vol. 4 (12), 370-3
- **MIFFLIN, ST JEOR et al.** A new predictive equation for resting energy expenditure in healthy individuals. *American Journal of Clinical Nutrition. 51, Nr. 2, Comparison of Predictive Equations for Resting Metabolic Rate in Healthy Nonobese and Obese Adults: A Systematic Review,* vol. 51 (2), 241-247